# EUROPEAN PATENT SPECIFICATION

(11) **EP 4 476 395 B1**
(45) Date of publication and mention of the grant of the patent: **06.05.2026**
(21) Application number: 23706676.6
(22) Date of filing: 08.02.2023
(51) Int. Cl.: A61L 2/10, D06M 10/00, D06B 19/00, D06B 3/12

(54) **REACTOR AND METHOD FOR TREATING TEXTILE FIBRES USING THE REACTOR**
REAKTOR UND VERFAHREN ZUR BEHANDLUNG VON TEXTILFASERN UNTER VERWENDUNG DES REAKTORS
RÉACTEUR ET PROCÉDÉ DE TRAITEMENT DES FIBRES TEXTILES UTILISANT LE RÉACTEUR

(30) Priority: 08.02.2022 IT 202200002219
(43) Date of publication of application: 18.12.2024
(73) Proprietor: Grinp S.R.L., 10036 Settimo Torinese (TO) (IT)
(72) Inventor: PARISI, Francesco, 10036 Settimo Torinese TO (IT)
(74) Representative: Perani & Partners S.p.A.
(86) International application number: PCT/IB2023/051108
(87) International publication number: WO 2023/152648

(56) References cited:
- CN-U- 209 361 384
- JP-A- H1 143 862
- KR-A- 20090 103 160
- US-A- 5 407 446

## Description

### Technical Field

The present invention relates to the field of production and treatment of textile fibres, preferably in the form of fabrics. In particular, the present invention focuses on a reactor for the treatment of textile fibres on a batch or continuous textile fibre production line capable of treating textile fibres downstream and/or upstream of further operations and treatments on the textile fibres.

### State of the Art

They are known reactors for the treatment of textile fibres integrated on batch or continuous production lines which are intended to receive textile fibres and keep them wet for subsequent treatments and/or prevent their movement from causing damage. Specifically, known reactors introduce water vapour into a chamber as the textile fibres pass through so that they are at the temperature required for the reagents, previously applied on the fibre by means of an impregnation bath, to act.

Document US 5 407 446 A discloses a reactor for the treatment of textile fibres suitable for a textile fibre production line, wherein the reactor comprises a control chamber defining a control volume and comprising an inlet opening to the control volume for receiving textile fibres from the production line, and an outlet opening from the control volume for ejecting textile fibres onto the production line; movement means arranged within the control chamber in the control volume and configured to transport the textile fibres from the inlet opening to the outlet opening, input means configured to introduce into the control volume a textile fibre treatment substance, and one or more UV irradiation devices configured to emit UV rays into the control chamber to irradiate the textile fibres transported by the movement means and the control volume.

### Problems of the Prior Art

Known reactors for the treatment of textile fibres have several disadvantages related to the time required to carry out the relevant treatments. In fact, known reactors require large amounts of water, reagents and energy in order to provide an acceptable product. The processes are very long and require large-size machinery. Furthermore, the use of reagents with which fabrics are impregnated at the reactor inlet, such as soda ash, increases the fragility of the fabric. Consequently, the known reactors do not allow for a complete and correct treatment of the textile fibres, causing defects and imperfections that could emerge in the finished product.

### Object of the Invention

The object of the invention is to implement a reactor for the treatment of textile fibres capable of overcoming the drawbacks of the prior art mentioned above.

In particular, it is the object of the present invention to provide a reactor for the treatment of textile fibres capable of improving and optimising treatments on textile fibres by carrying out both superficial and in depth treatments in the fibre.

It is a further object of the present invention to provide a reactor capable of introducing a photo-reaction action for the treatment of textile fibres.

The defined technical task and the specified objects are substantially achieved by a reactor for the treatment of textile fibres comprising the technical characteristics set forth in one or more of the appended claims.

### Advantages of the invention

Advantageously, the reactor of the present invention makes it possible to treat textile fibres both superficially and in depth at the fibre level.

Advantageously, the reactor of the present invention makes it possible to treat textile fibres at 360°.

Advantageously, the reactor of the present invention makes it possible to sterilise textile fibres and to maintain the sterility of the environment where the treatment of textile fibres takes place.

Advantageously, the reactor of the present invention makes it possible to define an optimal environment for the treatment of textile fibres by also preparing them for further treatment.

Advantageously, the reactor of the present invention makes it possible to expose the fibres to less aggressive environments while reducing its fragility.

Advantageously, the reactor of the present invention allows chemical reactions to take place by photo-exposure.

Advantageously, the reactor of the present invention makes it possible to introduce a photo-reaction action into the textile process to improve the treatment of textile fibres as well as to increase the production speed and reduce final costs.

### BRIEF DESCRIPTION OF THE DRAWINGS

Further characteristics and advantages of the present invention will become more apparent from the approximate and thus non-limiting description of a preferred, but not exclusive, embodiment of a reactor for the treatment of textile fibres, as shown in the accompanying drawings:
- Figure 1 shows a schematic view of a reactor for the treatment of textile fibres according to a first embodiment of the present invention;
- Figure 2 shows a schematic view of a reactor for the treatment of textile fibres according to a second embodiment of the present invention.

### DETAILED DESCRIPTION

Even when not explicitly highlighted, the individual features described with reference to the specific embodiments must be considered as accessories and/or exchangeable with other features, described with reference to other embodiments.

The present invention relates to a reactor for the treatment of textile fibres globally referred to as 1 in the Figures.

The reactor for the treatment of textile fibres 1 is configured to treat textile fibres both superficially and in depth while increasing the speed of the treatments and reducing water and energy waste. Preferably, the reactor 1 is configured to act on each fibre and in the environment wherein the textile fibres are moved within the reactor 1. More preferably, the reactor 1 may act directly and/or indirectly by activating reagents/solutions present in/on the textile fibre and/or in the environment wherein the textile fibre is moved.

According to a preferred embodiment, the reactor 1 is configured to define an environment for the treatment of textile fibres that optimises the treatment thereof and/or prepares them for subsequent treatments and operations along the production line.

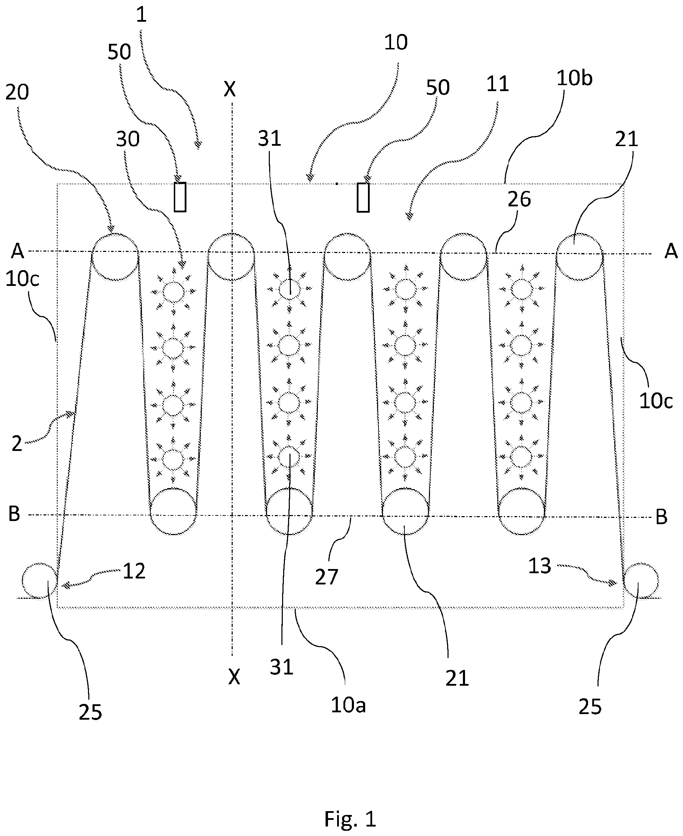

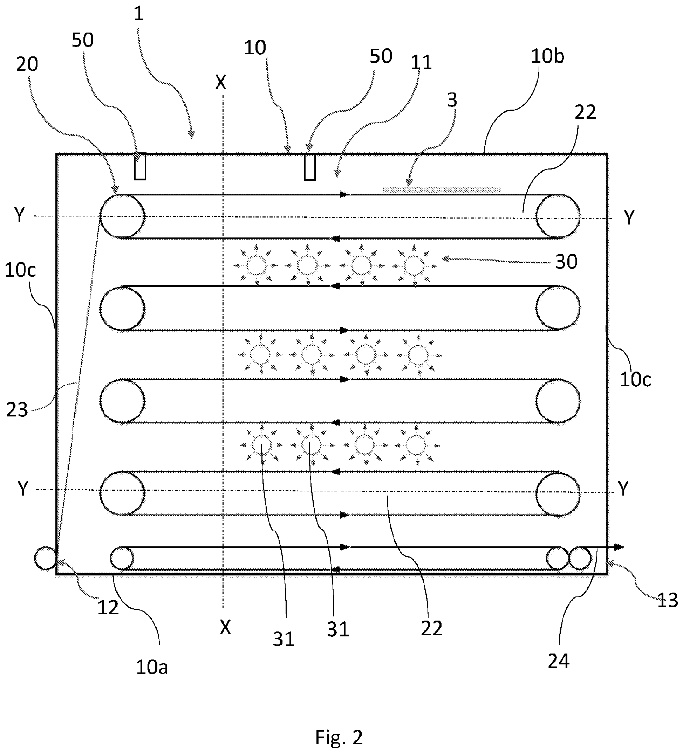

For the purposes of the present invention, textile fibres refer to shuttle-woven or knitted textile fibres of different materials such as cotton, polyesters, mixed fibres, viscose and continuous wool. As explained hereinafter, shuttle-woven and knitted textile fibres differ in the way they are transported along the production line in that the former can be stretched (Fig. 1), while the latter must be dragged/transported (Fig.2) reducing tension thereof as much as possible to avoid damage.

Preferably, the textile fibres are woven together in ways known to the person skilled in the art to define a fabric. Specifically, the textile fibre, and therefore the fabric, has a sheet-like shape in which two opposite surfaces are spaced out by a small thickness compared to the extension of the other length and width magnitudes.

According to an embodiment, the reactor 1 may be associated to a continuous textile fibre production line by arranging it along the continuous production line. According to an alternative embodiment, the reactor 1 may be associated to a textile fibre batch production line by arranging it in a station so that an operator loads the textile fibres according to the treatment to be performed.

The reactor 1 comprises a control chamber 10. Preferably, the control chamber 10 is configured to receive textile fibres from the production line. Specifically, the control chamber 10 defines a control volume 11 where the treatment of textile fibres can take place. Preferably, as explained hereinafter, the control chamber 10 is configured to define a controlled atmosphere within the control volume 11 for the treatment of textile fibres and any subsequent processing and treatment.

The control chamber 10 comprises an inlet opening 12 to the control volume 11 for receiving textile fibres from the production line and an outlet opening 13 from the control volume 11 for ejecting textile fibres onto the production line. Specifically, the control chamber 10 is configured to receive textile fibres to be treated from the inlet opening 12 and to eject the treated textile fibres from the outlet opening 13. Preferably, the inlet 12 and outlet 13 openings are also configured to handle the controlled atmosphere in the control volume 11.

It is worth noting that textile fibres can enter the control volume 11 impregnated with one or more substances, such as a solution containing a reagent and/or water. In an alternative embodiment, which may be combined with the previous one, the textile fibres can enter the control volume 11 as dry.

Preferably, further substances may be introduced into the control chamber 10 and dispersed in the control volume 11 and/or deposited by precipitation on the textile fibres in the control volume 11. It is thereby possible to control the substances on the textile fibres and in the control volume 11 by directly introducing substances into the control volume 11. According to preferred embodiments, these substances may be activated and/or made to react with each other, as hereinafter explained, by UV rays. It is worth noting that these reactions and activations are further optimised by maintaining the controlled atmosphere in the control volume 11.

Preferably, the control chamber 10 comprises a base 10a and a cover 10b spaced from the base 10a along a spacing direction X-X transverse to the base 10a and to the cover 10b preferably perpendicular to the base 10a and to the cover. The control room 10 comprises a side wall 10c configured to connect the base 10a and the cover 10b and defines together with the base 10a and the cover 10b the control volume 11. It is worth noting that according to the preferred embodiments shown in the Figures, the inlet opening 12 and the outlet opening 13 are obtained on the side wall 10c.

The reactor 1 comprises movement means 20 arranged within the control chamber 10 in the control volume 11. These movement means 20 are configured to transport the textile fibres from the inlet opening 12 to the outlet opening 13. Preferably, the movement means 20 allow to transport the textile fibres depending on the treatment to be carried out on the fibres and depending on the type of fibre. Specifically, the movement means 20 are configured to alternately expose both of the surfaces of the textile fibre to improve the treatment on the textile fibre and, as explained hereinafter, to improve exposure thereof to UV rays.

According to a preferred embodiment shown in Fig. 1, the movement means 20 comprise motorised rollers 21 configured to keep the textile fibres of the shuttle-woven type in tension. Specifically, the movement means 20 comprise a plurality of motorised rollers 21 to create a substantially zigzag path for the textile fibre. In this case, in order to maintain the tension, the movement means 20 comprise tensioning means 25 outside the control chamber 10 and associated to the production line. These tensioning means 25 are configured to apply opposite forces to maintain the tension and at the same time allow the movement of the textile fibre from the inlet opening 12 to the outlet opening 13. Specifically, motorised rollers 21 are arranged adjacent to each other along a first row 26 next to the cover 10b and a second row 27 next to the base 10a. It is worth noting that the first row 26 and the second row 27 are spaced along the spacing direction X-X. Each motorised roller 21 of each row is spaced from the adjacent roller respectively along a first direction A-A and along a second direction B-B substantially parallel to each other. In detail, the motorised rollers 21 of the first row 26 are staggered with respect to the motorised rollers 21 of the second row 27, so that a motorised roller 21 of the second row 27 is arranged at the spacing between two motorised rollers 21 of the first row 26. Thereby, the textile fibre passes alternately from a motorised roller 21 of the first row 26 to a motorised roller 21 of the second row 27 winding itself at least partly around the respective motorised rollers 21 until it exits the control volume 11. In Fig. 1 the textile fibre, referred to as 2, is shown schematically and in side view.

Alternatively, according to the preferred embodiment of Fig. 2 the movement means 20 comprise belts 22 configured to move knitted textile fibres while avoiding applying excessive tension thereon. In detail, the belts 22 provide a conveyor belt wound around two rollers configured to move the conveyor belt around the rollers. Preferably, the belts 22 are spaced along the spacing direction X-X parallel to each other and parallel to the base 10a between the cover 10b and the base 10a. Each belt 22 is configured to move the textile fibre along a movement direction Y-Y. Specifically, the belts 22, in the order from the one next to the cover to the one next to the base, alternately move the textile fibre along opposite movement directions. In detail, the belt 22 next to the cover 10b moves the textile fibre in the direct movement direction Y-Y from the inlet opening 12 to the outlet opening 13 while the subsequent belt 22 moves the textile fibre in the direct movement direction Y-Y from the outlet opening 13 to the inlet opening 12 and so on up to the belt 22 next to the base which directs the textile fibre towards the outlet opening 13. The movement directions are, for example, indicated with the arrows in Fig.2. Thereby, the textile fibres are transported by the belt 22 next to the cover 10b to the belt 22 next to the base 10a, causing them to precipitate by gravity from one belt 22 to the next, making a substantially zigzag movement. In this case, the movement means 20 comprise an inlet belt 23 and an outlet belt 24 connected respectively to the belt next to the cover and the belt next to the base 10a. Thereby, the textile fibre is transported from the inlet opening 12 to the belt next to the cover by the inlet belt 23, while it is ejected from the chamber by the outlet belt 24. In Fig. 2 the textile fibre, referred to as 3, is shown schematically and in side view.

It is worth noting that the motorised rollers 21 and belts 22 are known in the state of the art for moving shuttle-woven and knitted textile fibres respectively, and can therefore be used in embodiments alternative to those described above.

The reactor 1 comprises one or more UV irradiation devices 30 configured to emit UV rays into the control chamber 10. Specifically, the irradiation devices are configured to irradiate the textile fibres transported by the movement means 20 and the control volume 11 by means of UV rays (schematically indicated by arrows extending from the irradiation devices 30). In detail, the UV rays emitted by the irradiation devices 30 are dispersed in the control volume 11 by irradiating its contents, i.e. the space inside the control volume 11 and the textile fibres themselves.

According to the invention, the irradiation devices 30 are configured to irradiate, by UV rays, substances for the treatment of textile fibres introduced into the control volume 11. Specifically, UV rays can act on the substances, such as reagents, to activate them and/or promote chemical reactions, specifically photo reactions.

Advantageously, the use of UV rays makes it possible to act both superficially and in depth in the fibre. In fact, UV rays can pass through the textile fibres, promoting sterilisation of both the textile fibre and the control volume and, if reagents are present, activate them both on the surface and in depth, as well as promoting reactions between reagents in the control volume, on the surface of the textile fibres and in depth.

Advantageously, the use of UV rays makes it possible to generate active species such as radicals and/or oxidants that can act deep down in the fibre, thus improving the treatment.

Advantageously, the use of UV rays makes it possible to speed up the treatment of the textile fibres by increasing the production rate and at the same time reducing at least partly the use of water required to keep the fabric wet. In fact, UV rays are able to quickly activate the substances introduced into the control volume 11 and promote reactions between these substances in an optimised way.

Preferably UV-ray irradiation devices 30 are configured to irradiate the textile fibres and control volume 11 at 360°. Specifically, the movement means make it possible to expose both the surfaces of the textile fibres to the treatment and the UV rays emitted can pass through the textile fibres, acting both superficially and in depth. Textile fibres can thereby be treated comprehensively. According to a preferred embodiment, the irradiation devices 30 are arranged between the movement means 20. Specifically, the irradiation devices 30 are arranged between the movement means 20 between the turns of the zigzags in which the textile fibres are moved so as to optimise UV ray exposure.

According to the embodiments shown in the Figures, as an example, the irradiation devices 30 are arranged between the first 26 and second 27 rows along the spacing direction X-X between the motorised rollers in the first row (Fig. 1) or between two adjacent belts 22 (Fig.2).

According to a preferred embodiment, each UV irradiation device 30 is configured to emit UV rays of the UV-A, UV-B, UV-C type or a combination thereof with a wavelength comprised between 100nm and 400nm.

Preferably, each UV-ray emitting device 30 comprises a UV lamp 31. More preferably, the UV lamps 31 can be cylindrical or rectangular in cross-section, LED or vapour lamps.

According to a preferred embodiment, the reactor comprises irradiation control means in signal communication with each UV irradiation device 30 and with the movement means 20 and configured to control the emission of UV rays onto the textile fibres and within the control volume 11. Irradiation control means are configured to optimise the treatment and exposure of the textile fibre to UV rays. Preferably, the irradiation control means together with the configuration of the movement means 20 and irradiation devices 30 make it possible to expose the textile fibres to the UV light and to remain inside the control chamber 10 for the time required for the treatment, e.g. by maintaining a constant fabric feed rate.

Thanks to the irradiation control means, each irradiation device 30 may be activated separately from the others in order to regulate the energy supplied to the textile fibre and/or the control volume 11. This allows not to expose to excessive radiation textile fibres that may be sensitive to high UV frequencies.

According to the invention, the reactor comprises input means 50 configured to introduce one or more substances comprising saturated water vapour and reagents into the control volume 11 to treat the textile fibres. Specifically, as mentioned above, the substances for the treatment of textile fibres are introduced directly into the control volume 11 by means of these input means 50. These substances are configured to remain partly dispersed in the control volume 11 and partly precipitate onto the textile fibre, impregnating it at least partly. It is worth noting that these substances make it possible to treat the textile fibre and, depending on the treatment, can be activated by UV rays prior to deposition thereof by precipitation on the textile fibre and/or following deposition thereof. In addition, these substances can react with other substances on or in the textile fibre by UV rays. In addition, these substances can be configured to maintain certain humidity and temperature conditions within the control volume 11 in order to promote treatments on textile fibres. Preferably, the reagents introduced may include chemical species that functionalise the textile fibre and make it possible to obtain treatments useful for the textile fibre, both for the preparation and the finishing.

According to a preferred embodiment, the input means 50 comprise one or more nozzles for introducing the relevant substances. Preferably, the input means 50 may be arranged near the cover 10b (as shown in the Figures), the base 10a, the side wall 10c, in the middle of the control volume 11 or in a combination thereof. It is worth noting that the arrangement of the input means 50 is a function of the treatment to be performed, the type of textile fibres and the atmosphere to be reached in the control volume 11. Preferably, the inlet opening 12 and outlet opening 13 can be used as explained hereinafter to regulate the concentration of substances within the control volume.

According to the preferred embodiment, the UV irradiation devices 30 are configured to activate, by irradiating, one or more reagents that are dispersed in the control volume 11 prior to precipitation thereof on the textile fibre and/or deposited by precipitation on the textile fibre and/or impregnated in the textile fibre supplied to the inlet opening 12 of the control chamber 10. Specifically, the irradiation devices 30 by means of UV rays make it possible to activate substances, such as reagents, present in the control volume 11 in a form dispersed in the volume, precipitated on the textile fibre and/or with which the textile fibre was impregnated before entering the control chamber 10. A treatment can thereby be performed on the textile fibre.

According to an embodiment alternative and combinable with the previous one, the UV irradiation devices 30 are configured to induce, by irradiation, a reaction between one or more reagents introduced into the control volume 11 by means of the input means 50 with substances impregnating the textile fibre supplied to the inlet opening 12 of the control chamber 10.

Thereby, UV rays induce photo reactions with and between the substances in the control volume 11.

For example, if the reagents are present in the control volume in a dispersed form, the purpose of UV rays is to activate them before they reach the textile fibre and the relative surface to be treated. If necessary, UV rays allow the reagents dispersed in the control volume to react with the substances (such as other reagents) with which the textile fibres are impregnated. In another case, UV rays directly activate the reagents on the surface within the textile fibre, treating it accordingly.

It is worth noting that the UV rays used enable a deep oxidation of the fibre by activating the substances introduced into the chamber, e.g. hydroxyl species. This activation occurs by the interaction of UV rays with the chemistry present in control volume 11, i.e. with substances dispersed and/or present on and in the tissue.

According to a preferred embodiment, the control chamber 10 together with the input means 50 are configured to regulate the temperature and humidity within the control volume 11 for the treatment of the textile fibre. Specifically, the control chamber 10 with the input means define a controlled atmosphere in the control volume 11. Advantageously, the controlled atmosphere as well as temperature and humidity promote the treatment of the textile fibre and the related reactions and/or activation of reagents and substances with UV rays.

It is worth noting that the controlled atmosphere is controlled by introducing and ejecting saturated water vapour via the inlet means 50 and the inlet 12 and outlet 13 openings. According to the invention, treatment reagents are also dispersed in the saturated water vapour while ensuring a controlled atmosphere.

According to the invention, vapour is used both to heat the control chamber 10 and to allow the optimal treatment temperature to be reached, and to keep the treated fabric wet. In addition, since the vapour used is saturated, it is possible to obtain an accurate temperature control. Vapour allows the fabric not to dry out and to remain wet for as long as UV reactions and activations take place.

Preferably, the vapour introduced into the control chamber 10 is used to prevent the fabric from drying out so as to allow the UV rays to react with the other substances introduced and/or present inside the chamber, preferably deposited on the fabric, preventing these substances from evaporating and losing their effect, thus giving them time to react with the fabric.

According to the invention, the combination of saturated water vapour introduced in a controlled manner and UV radiation makes it possible to define an optimal oxidising atmosphere by avoiding the evaporation of substances, preferably impregnating the fabric, and releasing all the oxidising energy of these substances to the fabric. In addition, this combination makes it possible to enrich the vapour in the chamber with hydroxyl groups that create a reactant atmosphere around the fabric.

It is worth noting that the use of UV rays makes it possible to sterilise the treated fabric and maintain the sterility of the reaction environment.

According to a preferred embodiment, the control chamber 10 comprises a set of sensors configured to detect the temperature and humidity within the control volume 11. This set of sensors 60 is of a type known to the person skilled in the art. In addition, the control chamber comprises atmospheric control means in signal communication with the set of sensors and input means 50. Atmosphere control means are configured to regulate the temperature and humidity by controlling the introduction of one or more substances chosen from water vapour, gas, nebulised solution or a combination thereof according to a comparison between a predefined temperature and humidity with the detected temperature and humidity. As mentioned above, the atmosphere control means also make it possible to control the reagents to be introduced into the control chamber 11 for the treatment together with the water vapour, gas, atomised solution or alone. Advantageously, the atmosphere control means 60 make it possible to control in an automated way the controlled atmosphere within the control chamber according to the desired treatment.

An example of a treatment that can be obtained with the reactor of the present invention is bleaching. Specifically, the UV rays are intended to activate the various reagents, both those in the control volume 11 and those on the textile fibre, and enable a better treatment performance. The reagents adhering to the surface of the treated textile fibre are exposed to UV rays. These UV rays cause the reagents to be transformed into active oxidising species that act directly on the impurities in the fibre and make it possible to remove them in a subsequent rinse, and break down the naturally occurring dye compounds in the case of biogenic fibres. As a further example, the reactor of the present invention may be used for finishing.

A method for treating textile fibres using the reactor described above is a further object of the present invention. According to the invention, the method is part of a textile fibre production process and is carried out by the reactor 1 which is part of the relevant production line depending on the type of textile fibre. As will be clear from the sequence of steps, these may be carried out in parallel, with a degree of delay and/or anticipation with respect to the subsequent and/or previous steps.

Specifically, the method comprises a step of introducing one or more textile fibres into the control volume 11. Preferably, the introduction occurs by the inlet opening 12 and subsequent movement by means of movement means 20. As mentioned above, the fibres introduced may be dry or impregnated with treatment substances and/or water.

The method according to the invention comprises a step of introducing substances comprising saturated water vapour and reagents for treating the textile fibres into the control volume. This introduction can take place via input means 50 as described above. According to an alternative embodiment that can be combined with the previous one, the substances for treating textile fibres can also be introduced, at least in part, by introducing via the inlet opening 12 a fabric previously impregnated by means of relative impregnation means.

Preferably, the method makes it possible to provide the substances needed for the treatment quickly and efficiently. According to a preferred embodiment, the method also comprises a step of regulating the atmosphere inside the control volume 11 by means of the input means 50 and the inlet 12 and outlet 13 openings. Advantageously, the controlled atmosphere in the control volume in temperature, humidity and preferably in substances for the treatment of textile fibres makes it possible to improve the treatment while reducing waste.

The method comprises a step of activating, by irradiating UV rays, one or more reagents dispersed in the control volume 11 prior to precipitation thereof on the textile fibre and/or deposited by precipitation on the textile fibre and/or impregnated in the textile fibre supplied to the inlet opening 12 of the control chamber 10. According to an embodiment alternative and combinable with the previous one, the method comprises a step of inducing by irradiation a reaction between one or more reagents introduced into the control volume 11 by means of the input means 50 with substances impregnating the textile fibre supplied to the inlet opening 12 of the control chamber 10.

Specifically, the steps of activating and/or inducing a reaction by means of the UV rays emitted by the relative irradiation devices 30 make it possible to speed up and optimise the treatment of textile fibres. It is worth noting that the method may include a step of sterilising the control chamber 10 that can be combined with the previous steps.

The method comprises a step of removing the treated textile fibres from the control volume 11.

It must to be noted that the movement and maximum exposure to treatment is facilitated and implemented by the movement means 20 that transport the textile fibres from the inlet opening 12 to the outlet opening 13 while carrying out the introduction to removal steps.

## Claims

1. A reactor for the treatment of textile fibres (1) for a textile fibre production line, the reactor for the treatment of textile fibres (1) comprising:
- a control chamber (10) defining a control volume (11) and comprising an inlet opening (12) to the control volume (11) for receiving textile fibres (1) from the textile fibre production line and an outlet opening (13) from the control volume (11) for ejecting textile fibres (1) onto the textile fibre production line;
- movement means (20) arranged within the control chamber (10) in the control volume (11) and configured to transport the textile fibres (1) from the inlet opening (12) to the outlet opening (13);
wherein the reactor further comprises:
- input means (50) configured to introduce into the control volume (11) one or more textile fibre treatment substances comprising saturated water vapour and reagents configured to remain partly dispersed in the control volume (11) and to precipitate at least partly onto the textile fibres (1);
- one or more UV irradiation devices (30) configured to emit UV rays into the control chamber (10) to irradiate the textile fibres (1) transported by the movement means (20), the control volume (11) and substances introduced into the control volume (11) for the treatment of the textile fibres (1).

2. The reactor (1) according to claim 1, wherein the UV irradiation devices (30) are configured to irradiate the textile fibres (1) and the control volume (11) at 360°.

3. The reactor (1) according to claim 1 or 2, wherein each UV irradiation device (30) is configured to emit UV rays of the UV-A, UV-B, UV-C type or a combination thereof with a wavelength comprised between 100nm and 400nm.

4. The reactor (1) according to any one of claims 1 to 3, wherein each UV irradiation device (30) comprises a UV lamp (31).

5. The reactor (1) according to any one of claims 1 to 4, wherein it comprises irradiation control means in signal communication with each UV irradiation device (30) and with the movement means (20) and configured to control the emission of UV rays onto the textile fibres (1) and within the control volume (11).

6. The reactor (1) according to any claims 1 to 5, wherein the UV irradiation devices (30) are configured to:
- activate by irradiating one or more reagents dispersed in the control volume (11) prior to precipitation on the textile fibres (1) and/or deposited by precipitation on the textile fibres (1) and/or impregnated in the textile fibres (1) supplied to the inlet opening (12) of the control chamber (10); and/or
- induce by irradiation a reaction between one or more reactants introduced into the control volume (11) by means of the input means (50) with substances impregnating the textile fibres (1) supplied to the inlet opening (12) of the control chamber (10).

7. The reactor (1) according to any claims 1 to 6, wherein the control chamber (10) together with the input means (50) are configured to regulate the temperature and humidity within the control volume (11) for the treatment of the textile fibres (1) by defining a controlled atmosphere in the control volume (11) by the inlet and ejection of saturated water vapour via the input means (50) and the inlet (12) and outlet (13) openings.

8. The reactor (1) according to claim 7, wherein the control chamber (10) comprises:
- a set of sensors configured to detect the temperature and humidity within the control volume (11);
- atmosphere control means in signal communication with the set of sensors and the input means (50) and configured to regulate the temperature and humidity by controlling the inlet of saturated water vapour according to a comparison between a predefined temperature and humidity with the detected temperature and humidity.

9. A method for the treatment of textile fibres comprising the steps of:
- providing a reactor (1) according to claims 1 to 8;
- introducing into the control volume (11) one or more textile fibres (1) dry or impregnated with substances to treat the textile fibres (1);
- introducing into the control volume substances comprising saturated water vapour and reagents to treat the textile fibres (1) by means of the input means (50);
- activating by irradiating with UV rays one or more reagents dispersed in the control volume (11) prior to their precipitation on the textile fibres (1) and/or deposited by precipitation on the textile fibres (1) and/or impregnated in the textile fibres (1) supplied to the inlet opening (12) of the control chamber (10) and/or inducing by irradiation by means of UV rays emitted by the relative irradiation devices (30) a reaction between one or more reagents introduced into the control volume (11) by means of the input means (50) with substances impregnating the textile fibres (1) supplied to the inlet opening (12) of the control chamber (10);
- removing the treated textile fibres (1) from the control volume (11).

## Patentansprüche

1. Reaktor zur Behandlung von Textilfasern (1) für eine Textilfaser-Produktionslinie, wobei der Reaktor zur Behandlung von Textilfasern (1) umfasst:
o eine Steuerkammer (10), die ein Steuervolumen (11) definiert und eine Einlassöffnung (12) zum Steuervolumen (11) zur Aufnahme von Textilfasern (1) von der Textilfaser-Produktionslinie und eine Auslassöffnung (13) aus dem Steuervolumen (11) zum Ausstoßen der Textilfasern (1) auf die Textilfaser-Produktionslinie umfasst;
o Bewegungsmittel (20), die innerhalb der Steuerkammer (10) im Steuervolumen (11) angeordnet und so konfiguriert sind, dass sie die Textilfasern (1) von der Einlassöffnung (12) zur Auslassöffnung (13) transportieren; **dadurch gekennzeichnet, dass** der Reaktor ferner umfasst:
o Einbringmittel (50), die so konfiguriert sind, dass sie in das Steuervolumen (11) eine oder mehrere Textilfaser-Behandlungssubstanzen einbringen, die gesättigten Wasserdampf und Reagenzien umfassen und so konfiguriert sind, dass sie teilweise im Steuervolumen (11) dispergiert bleiben und zumindest teilweise auf den Textilfasern (1) ausfallen;
o eine oder mehrere UV-Bestrahlungsvorrichtungen (30), die so konfiguriert sind, dass sie UV-Strahlen in die Steuerkammer (10) emittieren, um die von den Bewegungsmitteln (20) transportierten Textilfasern (1), das Steuervolumen (11) und die in das Steuervolumen (11) zur Behandlung der Textilfasern (1) eingebrachten Substanzen zu bestrahlen.

2. Reaktor (1) nach Anspruch 1, wobei die UV-Bestrahlungsvorrichtungen (30) so konfiguriert sind, dass sie die Textilfasern (1) und das Steuervolumen (11) bei 360° bestrahlen.

3. Reaktor (1) nach Anspruch 1 oder 2, wobei jede UV-Bestrahlungsvorrichtung (30) so konfiguriert ist, dass sie UV-Strahlen des UV-A-, UV-B-, UV-C-Typs oder eine Kombination davon mit einer Wellenlänge zwischen 100 nm und 400 nm emittiert.

4. Reaktor (1) nach einem der Ansprüche 1 bis 3, wobei jede UV-Bestrahlungsvorrichtung (30) eine UV-Lampe (31) umfasst.

5. Reaktor (1) nach einem der Ansprüche 1 bis 4, wobei er Bestrahlungs-Steuermittel umfasst, die in Signalkommunikation mit jeder UV-Bestrahlungsvorrichtung (30) und mit den Bewegungsmitteln (20) stehen und so konfiguriert sind, dass sie die Emission von UV-Strahlen auf die Textilfasern (1) und innerhalb des Steuervolumens (11) steuern.

6. Reaktor (1) nach einem der Ansprüche 1 bis 5, wobei die UV-Bestrahlungsvorrichtungen (30) konfiguriert sind zum:
o Aktivieren, durch Bestrahlen, eines oder mehrerer im Steuervolumen (11) dispergierter Reagenzien vor deren Ausfallen auf den Textilfasern (1) und/oder die durch Ausfallen auf den Textilfasern (1) abgelagert sind und/oder die in den Textilfasern (1) imprägniert sind, die der Einlassöffnung (12) der Steuerkammer (10) zugeführt werden; und/oder
∘ Induzieren, durch Bestrahlen, einer Reaktion zwischen einem oder mehreren mittels der Einbringmittel (50) in das Steuervolumen (11) eingebrachten Reaktanten mit Substanzen, die die Textilfasern (1) imprägnieren, die der Einlassöffnung (12) der Steuerkammer (10) zugeführt werden.

7. Reaktor (1) nach einem der Ansprüche 1 bis 6, wobei die Steuerkammer (10) zusammen mit den Einbringmitteln (50) so konfiguriert ist, dass sie die Temperatur und die Feuchtigkeit innerhalb des Steuervolumens (11) für die Behandlung der Textilfasern (1) reguliert, indem eine kontrollierte Atmosphäre im Steuervolumen (11) durch das Einleiten und Ausstoßen von gesättigtem Wasserdampf über die Einbringmittel (50) und die Einlass- (12) und Auslass- (13) Öffnungen definiert wird.

8. Reaktor (1) nach Anspruch 7, wobei die Steuerkammer (10) umfasst:
∘ einen Satz von Sensoren, die so konfiguriert sind, dass sie die Temperatur und die Feuchtigkeit innerhalb des Steuervolumens (11) erfassen;
o Atmosphären-Steuermittel, die in Signalkommunikation mit dem Satz von Sensoren und den Einbringmitteln (50) stehen und so konfiguriert sind, dass sie die Temperatur und die Feuchtigkeit durch Steuern des Einlasses von gesättigtem Wasserdampf gemäß einem Vergleich zwischen einer vordefinierten Temperatur und Feuchtigkeit und der erfassten Temperatur und Feuchtigkeit regulieren.

9. Verfahren zur Behandlung von Textilfasern, umfassend die Schritte:
∘ Bereitstellen eines Reaktors (1) nach den Ansprüchen 1 bis 8;
∘ Einbringen einer oder mehrerer trockener oder mit Substanzen zur Behandlung der Textilfasern (1) imprägnierter Textilfasern (1) in das Steuervolumen (11);
o Einbringen von Substanzen, die gesättigten Wasserdampf und Reagenzien umfassen, zur Behandlung der Textilfasern (1) in das Steuervolumen mittels der Einbringmittel (50);
o Aktivieren, durch Bestrahlen mit UV-Strahlen, eines oder mehrerer im Steuervolumen (11) dispergierter Reagenzien vor deren Ausfallen auf den Textilfasern (1) und/oder die durch Ausfallen auf den Textilfasern (1) abgelagert sind und/oder die in den Textilfasern (1) imprägniert sind, die der Einlassöffnung (12) der Steuerkammer (10) zugeführt werden, und/oder Induzieren, durch Bestrahlen mittels von den entsprechenden Bestrahlungsvorrichtungen (30) emittierten UV-Strahlen, einer Reaktion zwischen einem oder mehreren mittels der Einbringmittel (50) in das Steuervolumen (11) eingebrachten Reagenzien mit Substanzen, die die Textilfasern (1) imprägnieren, die der Einlassöffnung (12) der Steuerkammer (10) zugeführt werden;
o Entnehmen der behandelten Textilfasern (1) aus dem Steuervolumen (11).

## Revendications

1. Réacteur pour le traitement de fibres textiles (1) pour une ligne de production de fibres textiles, le réacteur pour le traitement de fibres textiles (1) comprenant :
∘ une chambre de commande (10) définissant un volume de commande (11) et comprenant une ouverture d'entrée (12) vers le volume de commande (11) pour recevoir des fibres textiles (1) depuis la ligne de production de fibres textiles et une ouverture de sortie (13) depuis le volume de commande (11) pour éjecter les fibres textiles (1) sur la ligne de production de fibres textiles ;
∘ des moyens de déplacement (20) agencés à l'intérieur de la chambre de commande (10) dans le volume de commande (11) et configurés pour transporter les fibres textiles (1) de l'ouverture d'entrée (12) à l'ouverture de sortie (13) ; dans lequel le réacteur comprend en outre :
∘ des moyens d'introduction (50) configurés pour introduire dans le volume de commande (11) une ou plusieurs substances de traitement de fibres textiles comprenant de la vapeur d'eau saturée et des réactifs, configurées pour rester partiellement dispersées dans le volume de commande (11) et pour précipiter au moins partiellement sur les fibres textiles (1) ;
∘ un ou plusieurs dispositifs d'irradiation UV (30) configurés pour émettre des rayons UV dans la chambre de commande (10) afin d'irradier les fibres textiles (1) transportées par les moyens de déplacement (20), le volume de commande (11) et les substances introduites dans le volume de commande (11) pour le traitement des fibres textiles (1).

2. Réacteur (1) selon la revendication 1, dans lequel les dispositifs d'irradiation UV (30) sont configurés pour irradier les fibres textiles (1) et le volume de commande (11) à 360°.

3. Réacteur (1) selon la revendication 1 ou 2, dans lequel chaque dispositif d'irradiation UV (30) est configuré pour émettre des rayons UV du type UV-A, UV-B, UV-C ou une combinaison de ceux-ci avec une longueur d'onde comprise entre 100 nm et 400 nm.

4. Réacteur (1) selon l'une quelconque des revendications 1 à 3, dans lequel chaque dispositif d'irradiation UV (30) comprend une lampe UV (31).

5. Réacteur (1) selon l'une quelconque des revendications 1 à 4, dans lequel il comprend des moyens de commande d'irradiation en communication de signal avec chaque dispositif d'irradiation UV (30) et avec les moyens de déplacement (20), et configurés pour commander l'émission de rayons UV sur les fibres textiles (1) et à l'intérieur du volume de commande (11).

6. Réacteur (1) selon l'une quelconque des revendications 1 à 5, dans lequel les dispositifs d'irradiation UV (30) sont configurés pour :
o activer par irradiation un ou plusieurs réactifs dispersés dans le volume de commande (11) avant leur précipitation sur les fibres textiles (1) et/ou déposés par précipitation sur les fibres textiles (1) et/ou imprégnés dans les fibres textiles (1) amenées à l'ouverture d'entrée (12) de la chambre de commande (10) ; et/ou
o induire par irradiation une réaction entre un ou plusieurs réactifs introduits dans le volume de commande (11) au moyen des moyens d'introduction (50) avec des substances imprégnant les fibres textiles (1) amenées à l'ouverture d'entrée (12) de la chambre de commande (10).

7. Réacteur (1) selon l'une quelconque des revendications 1 à 6, dans lequel la chambre de commande (10) conjointement avec les moyens d'introduction (50) sont configurés pour réguler la température et l'humidité à l'intérieur du volume de commande (11) pour le traitement des fibres textiles (1) en définissant une atmosphère contrôlée dans le volume de commande (11) par l'introduction et l'éjection de vapeur d'eau saturée via les moyens d'introduction (50) et les ouvertures d'entrée (12) et de sortie (13).

8. Réacteur (1) selon la revendication 7, dans lequel la chambre de commande (10) comprend :
∘ un ensemble de capteurs configurés pour détecter la température et l'humidité à l'intérieur du volume de commande (11) ;
o des moyens de commande d'atmosphère en communication de signal avec l'ensemble de capteurs et les moyens d'introduction (50) et configurés pour réguler la température et l'humidité en commandant l'introduction de vapeur d'eau saturée selon une comparaison entre une température et une humidité prédéfinies et la température et l'humidité détectées.

9. Procédé de traitement de fibres textiles comprenant les étapes consistant à :
o fournir un réacteur (1) selon les revendications 1 à 8 ;
o introduire dans le volume de commande (11) une ou plusieurs fibres textiles (1) sèches ou imprégnées de substances pour traiter les fibres textiles (1) ;
o introduire dans le volume de commande des substances comprenant de la vapeur d'eau saturée et des réactifs pour traiter les fibres textiles (1) au moyen des moyens d'introduction (50) ;
o activer par irradiation avec des rayons UV un ou plusieurs réactifs dispersés dans le volume de commande (11) avant leur précipitation sur les fibres textiles (1) et/ou déposés par précipitation sur les fibres textiles (1) et/ou imprégnés dans les fibres textiles (1) amenées à l'ouverture d'entrée (12) de la chambre de commande (10) et/ou induire par irradiation au moyen de rayons UV émis par les dispositifs d'irradiation relatifs (30) une réaction entre un ou plusieurs réactifs introduits dans le volume de commande (11) au moyen des moyens d'introduction (50) avec des substances imprégnant les fibres textiles (1) amenées à l'ouverture d'entrée (12) de la chambre de commande (10) ;
∘ retirer les fibres textiles traitées (1) du volume de commande (11).
